# EUROPEAN PATENT APPLICATION

(11) **EP 1 629 859 A1**
(43) Date of publication of application: **01.03.2006**
(21) Application number: 05255292.4
(22) Date of filing: 26.08.2005
(51) Int. Cl.: A61M 16/06, A61M 16/10, A62B 9/00, A61M 16/08, A61M 16/20

(54) **Face mask**

(30) Priority: 26.01.2005 GB 0501558; 27.08.2004 GB 0419114
(71) Applicant: Talbot, John Storey, Derwen Fawr Swansea SA2 8DZ (GB)
(72) Inventor: Talbot, John Storey, Derwen Fawr Swansea SA2 8DZ (GB)
(74) Representative: Gallafent, Antony Xavier

(57) **Abstract**

The present invention relates to a face mask for alleviating the symptoms of mouth breathing. The invention is a face mask comprising a housing (2) being arranged and configured to fit over at least a user's mouth, the housing (2) defining a hollow space between the user's mouth and the inner surface of the housing (2). There is also provided a means (6) for removably holding the housing (2) adjacent a user's mouth. The housing also contains an aperture (4) and a means to adjust the size of the aperture to control the air flow into and out of the housing (2) such that the air temperature and/or humidity of the air in the mask can be controlled.

## Description

The present invention relates to an apparatus for alleviating the symptoms associated with mouth breathing, and in particular relates to a face mask for such purpose.

The common cold is one of the most widespread sicknesses with symptoms of headaches, coughing, sneezing, and in particular sore throats. Sore throats can be caused by a person being forced to breathe through their mouths, and is particularly acute in environments in which the air is dry such as in air-conditioned or centrally heated homes. The healthy nose has the effect of humidifying and warming the incoming air. Even if the nose is not completely blocked during an infection, the nose and throat are hypersensitive to dryness and cooling. Any breathing can cause pain, throaty cough and other unpleasant symptoms in the nose and throat, especially at night. During the daytime, the effect of dry and sore throats is treated by varying the mouth aperture, the use of salivation, drinking fluids and seeking humidity, however these become impossible when a person is asleep.

It is also common knowledge that symptoms can be relieved by cupping one's hands over the face, sleeping under bedclothes or using warmers and humidifiers, but none of these can be comfortably maintained for very long, particularly at night.

North American Indians used similar principles to treat the common cold and prevent its complications (George Catlin, Shut your mouth and save your life, 1891, p.XX11-XX111). Their methods included the rebreathing, warming and humidification of air. These days their methods however would be judged inconvenient, completely wrapping themselves in their Buffalo skin and breathing into cupped hands for several hours at the onset of symptoms.

More modem evidence suggests that correct airway temperature is important in terms of vulnerability to infection by viruses and of recovery. The viruses in question multiply best at 32 degrees centigrade, 5 degrees below core body temperature (37 degrees centigrade), such lower temperatures are likely to obtain quite a way into the respiratory system in cool weather.

The seasonality of infection has been accounted for by low environmental temperature (as opposed to crowding which occurs equally in summer). [Eccles, R (2002) "An explanation for the seasonality of acute upper respiratory infections". Acta Otolaryngologica (Stockholm) 122:183-191].

Furthermore, unconscious patients who are not intubated often mouth breathe with the consequent need for increased oral hygiene measures. A similar effect is apparent in patients who may be conscious but who have blocked nasal passages such as after injury or surgery or those with faulty mouth control, such as after a stroke when respiration may for these reasons result in prolonged mouth breathing causing severe mouth dryness, halitosis, discomfort, and the risk of infection. The distress caused may often not be expressed by the severely disabled subject.

Finally, people who are unfit or exercise in very cold or dry environments may suffer from an excessive amount of mucus secretion and painful airways until such time as they are fit enough to undertake exercise with their mouths closed for a more significant proportion of the time.

Various masks have been suggested with particular reference for a user exercising in cold environments, for example in US Patent 5884336 wherein a hygroscopic material is maintained at a position adjacent a user's mouth, such that the moisture in the air breathed out is captured by the material and returns the moisture to the dry cold air to be breathed in.

The present invention provides a significantly simpler arrangement to that described in US 5884336.

According to the present invention, there is provided a face mask comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, said face mask including means to adjust the size of said aperture to control the air flow into and out of said housing, such that, in use, the humidity and/or temperature of the air within said housing may be controlled.

Preferably, the housing is arranged and configured to fit over a user's nose and mouth.

The means to adjust the size of the aperture may comprise overlapping slats, or alternatively may comprise at least one flexible flap.

The means to hold the housing adjacent a user's face preferably comprises at least one resiliently elastic strap, however it is envisaged that any suitable fastener may be used. In one embodiment the means to hold the mask adjacent the face of a user may be a scarf.

The housing may be constructed of a flexible material, which provides a comfortable fit on the face of a user. Alternatively, the housing may be constructed of a substantially rigid material, in particular a plastic material may be used.

Beneficially, the mask is disposable, however may be constructed such that it is optionally washable and re-usable.

The mask may further comprise means to adjust the volume of air within said housing. In one embodiment, the volume adjusting means may comprise a bellows type structure. Alternatively, the volume adjusting means comprises a telescopic arrangement.

There are numerous advantages associated with the provision of a variable volume of the cavity. For example, the comfort and versatility may be increased, and each user may be able to set a cavity volume suitable for their specific requirements. It may also provide a basis for research into the development of optional cavity sizes. Furthermore, a variable cavity may optimise the administration of various agents through it.

There may be provided a second aperture in said housing, arranged and configured to receive means to provide a gas into said cavity. Alternatively, gas may be administered through the adjustable aperture. This provides the benefit that inhalable elements such as oxygen or nebulised medication may be administered. The means to provide a gas into the cavity should be approved by the relevant standards authority.

According to a second aspect of the present invention, there is provided headwear comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, said face mask including means to adjust the size of said aperture to control the air flow into and out of said housing, such that, in use, the humidity and/or temperature of the air within said housing may be controlled.

According to a third aspect of the present invention, there is provided a face mask, when in use for prevention of one or more symptoms of nose and/or mouth breathing, the face mask comprising a housing being arranged and configured to fit over at least a user's mouth, said housing defining, in use, a hollow space between at least a user's mouth and the inner surface of the housing, further comprising means for removably holding said housing adjacent to at least a user's mouth, said housing further containing an aperture therein, such that, in use, the humidity and/or temperature of the air within said housing may be increased relative to the surrounding atmosphere.

Preferably, the face mask includes means to adjust the size of the aperture to control the air flow into and out of said housing such that, in use, the humidity and/or temperature of the air within the housing may be controlled.

There is further provided a method of preventing one or more symptoms of nose and/or mouth breathing, the method comprising the steps of providing a face mask comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, such that, in use, the humidity and/or temperature of the air within said housing may be increased relative to the surrounding atmosphere. The method preferably further utilises means to adjust the size of the aperture to control the air flow into and out of the housing such that, in use, the humidity and/or temperature of the air within the housing may be controlled.

An exemplary embodiment of the present invention will now be described with reference to the accompanying drawings, in which:
Figure 1 shows a schematic plan view of a first exemplary embodiment of the present invention.
Figure 2 shows a schematic plan view of a second exemplary embodiment of the present invention.
Figure 3 shows a schematic plan view of the second exemplary embodiment of the present invention, showing an alternative aperture arrangement.
Figure 4 is a schematic plan view of the second exemplary embodiment of the present invention, showing a further alternative aperture arrangement.
Figure 5 shows a schematic side view of the second exemplary embodiment showing a further alternative aperture arrangement.
Figure 6 shows three different stages of adjustability of the size of aperture associated with the exemplary embodiment of the aperture arrangement as shown in Figure 5.
Figure 7 is a schematic diagram of an alternative use of the mask arrangement.
Figure 8 is a schematic diagram of the mask arrangement incorporated into a user's scarf.
Figure 9 is a schematic side view of an exemplary embodiment of the adjustability of the cavity of the mask.
Figure 10 is a schematic side view of an alternative embodiment of a mechanism for adjusting the cavity side of the mask.

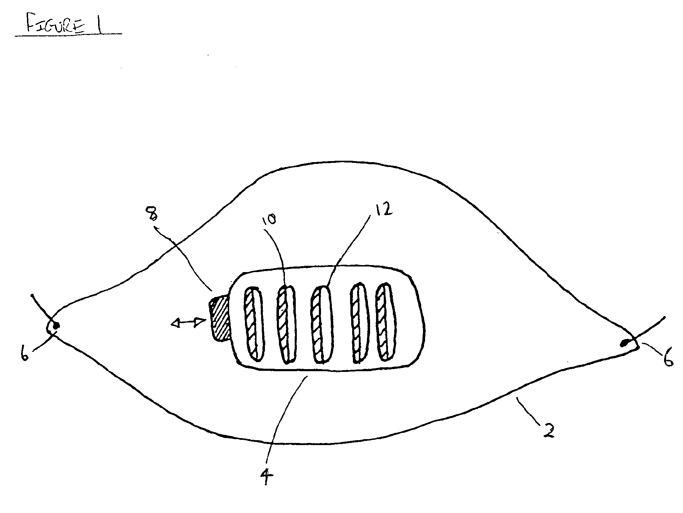

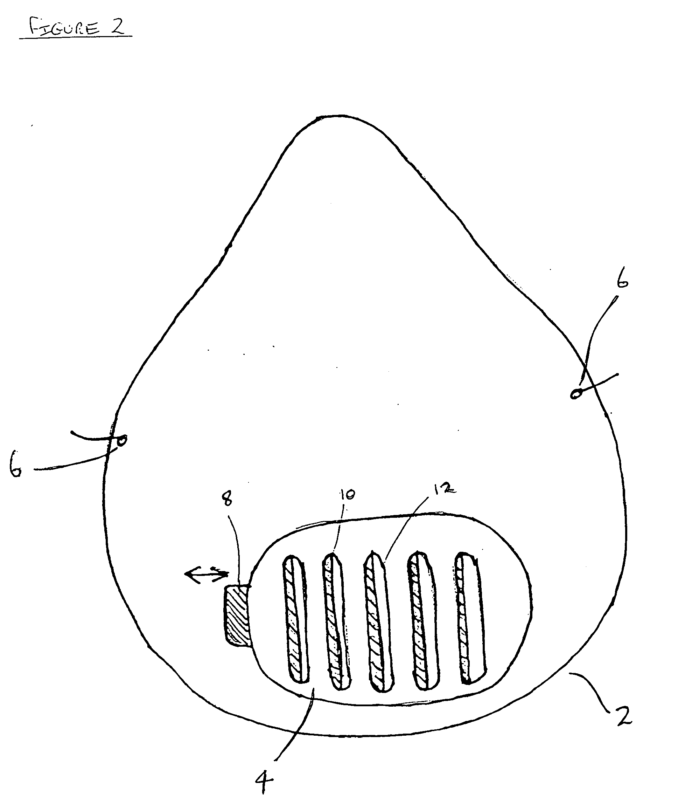

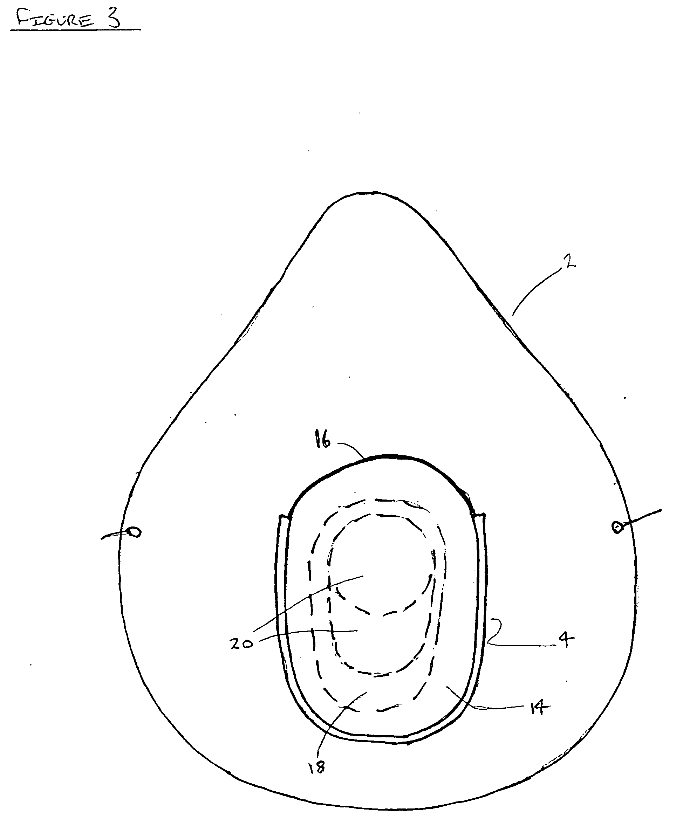

Referring to Figure 1, a housing 2 is shaped such that the mask fits creating a chamber around the user's mouth. The housing 2 is of a material sufficiently resilient that it will not crumple up with a users breath, or will remain substantially undeformed should it be knocked in use. The housing is shaped such that the fit is comfortable, and produces a secure fit between the edges of the housing 2 and the user's face. The adjustable aperture 4 is located in the housing 2 in the position adjacent the user's mouth, adjustable such that a large volume of fresh air may be taken in certain circumstances when the humidity and/or temperature external to the mask have increased. However, the aperture may be anywhere within the mask, or may even be at one edge of the mask, in the form of an adjustable cut-out from the mask edge. The aperture is generally positioned such that air is expelled downwards or to the side of a user's mouth to prevent steaming up of a user's glasses (if applicable). The fastening means 6 may be any known type, such as one or more stretched elastic straps for example. Other fastening means may be taping or clip straps, around the back of the head. Alternatively, the mask could be taped to the face, or elastic straps may be looped around a user's ears. The aperture is opened to the desired degree, as determined by the user or the user's carer such that a desired degree of humidity and warmth is achieved within the mask. Further examples of the aperture arrangement are shown in Figures 3 to 6, however in this embodiment an aperture consisting of movable overlapping slats is shown. A slider 8 is actuated by a user's fingers in the direction indicated by an arrow, which acts to push the solid portions of a sheet of slotted material 10 to be situated directly beneath the slots 12 in the surface of the outer sheet of the adjustable aperture 4. This allows the humidity within the mask housing 2 to be selectively controlled.

In this particular embodiment, the nose remains clear of the housing such that should the nose clear during the time when the user is asleep, then the user can automatically revert back to breathing normally through the nose.

The mask is made of a cheap material such as a plastic, and is re-usable and washable. Materials are chosen such that they will not be affected by any nasal secretions or a user's breath, and will also withstand regular washing. Ideally, the mask is sufficiently resiliently elastic such that it will not deform under normal use, but is sufficiently flexible such that the edges will deform to the shape of the face. In an alternative arrangement, the mask may be disposable after as few as one use, and therefore may be constructed of a cheaper disposable material such as a type of paper. The most suitable materials for construction of the mask,are in terms of insulating properties, moisture exchange, heat exchange, mask porosity and cavity variables in connection with the variable aperture range, and these may all be optimised for comfort and efficiency of the mask.

Referring to Figure 2, a preferred embodiment of the present invention is shown in which the user's nose and mouth are both covered by the mask. The advantage of this preferred embodiment provides is that as the nose gradually blocks and/or unblocks, the nasal passages remain hypersensitive to dryness and cooling and incomplete blockage can cause localised painful spots where the air is rushing past partial obstructions. In early stages of a cold and in other conditions, hypersensitivity of the nasal passages can occur without any physical blockage. The mask will, if covering the nose and the mouth, assist in these circumstances even if nasal breathing is still possible. Covering the nose maintains an increased humidity/warmth environment for nose breathing when this becomes possible. The housing 2 extends from the bridge of the nose via each cheek to the chin, and defines an air pocket between the inner surface of the housing 2 and the user's face. Again, it is possible that an adjustable aperture 4 is formed at one edge of the housing, however, in a preferred form the adjustable aperture 4 is formed adjacent the user's mouth. The adjustable aperture 4 may be any type of adjustable opening mechanism, including those described in Figures 1 to 6. The shape of the mask may be similar to that of a pilot's oxygen mask.

Only one or two respirations will be needed to return the temperature and humidity in the mouth, throat and partially unblocked passages to almost normal levels, via the warmth and humidity from within the chest passing through and residing in the mask, as it would in a normally functioning nasal cavity. Fine-tuning for comfort can then be varied at user discretion.

Alternative more complex chamber shapes and designs can be incorporated into the mask but are unlikely to be of added benefit to the simpler design unless after detailed airflow analysis they are found to reduce drafts or other anomalies in particular shapes of mask.

As well as the mark working by re-breathing of some of the previously exhaled warmed and humidified air, there would to a variable extent also be some heat and humidity exchange occurring on the inner surfaces of the mask along with the effects of insulation by the mask, of the contained air.

All these effects in combination in a light mask render complex apparatus unnecessary, especially when control and equilibrium of the mask's internal environment can be obtained by the adjustable aperture. They also make for a light convenient, self-contained and unencumbered mask.

If more research is undertaken into the materials to be used for the mask, the humidity exchange, the heat exchange and the insulation variables/properties could be tailored to optimise the mask's desired properties for the intended operating environment, from domestic to the most hostile conditions.

For example, at one extreme, higher insulation and humidity exchange properties could optimise the mask for use underneath conventional arctic head and neck clothing. Thus dispensing with cumbersome and weighty specialist air warmers whilst conferring the intended benefits and be light enough to carry on expeditions where any weight penalty is a disadvantage.

At the other end of the scale, the same is true of arid but hot conditions, such as deserts. For example, materials with high humidity exchange and low insulation, or even heat radiant properties would be optimal in such extreme conditions.

Each extreme again being fine-tuned by the adjustable control(s).

The variable aperture will also allow the appropriate fine control over comfort and optimal breathing in the commonest envisaged temperature range of a few degrees sub zero to upper room temperature; the best all round domestic use range.

For the more extreme conditions research into materials and best aperture range may be found to differ for specialist use in sub zero or hot arid environments from the domestic environment. It may be possible to develop a mask that can straddle all conditions.

Shown in Figure 3 is a plan view of a mask designed to cover both the mouth and nose, using an adjustable aperture of a multiple flap type arrangement which is designed such that varying size apertures may be used. The arrangement consists of a large flap 14 of a substantially rectangular shape, which is hinged at one edge 16 and overlaps the aperture 4 (not visible). The large flap 14 has an adhesive material around the edge of the surface area, such that it will stick to the housing 2 adjacent the aperture 4. A preferred method of adhesion is Velcro (RTM). The large flap 14 has within it a further aperture which is covered by a second smaller flap 18. Any number of similar flaps 20 and apertures of varying size may be included, however four flaps would be a suitable number to allow sufficient humidity and temperature adjustability within the mask. This arrangement allows the size of the aperture to be selected depending on the number of flaps that are open. They are held back by a further strip of Velcro (RTM) on the opposing edge to the hinged section. However, it will be appreciated that any other mechanism for adjusting the size or area of the aperture 4 can be used.

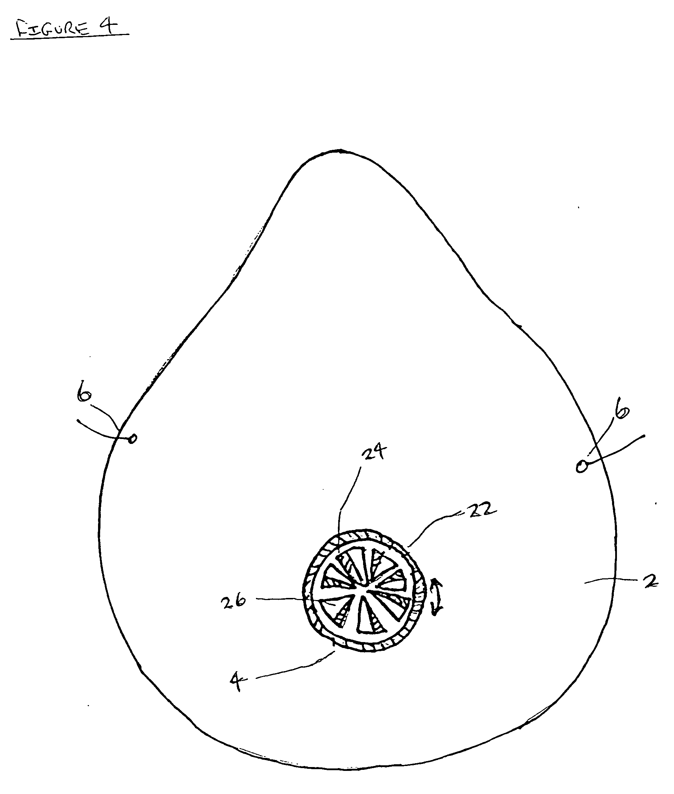

Figure 4 is a schematic plan view of a further mechanism for adjusting the aperture size, in a manner similar to that shown in Figures 1 and 2. However, in this embodiment, a rotating disc 22 is actuated by a user's fingers in the direction indicated by an arrow, which acts to push the solid portions of a sheet of slotted material 24 to be situated directly above the slots 26 in the surface of the outer sheet of the adjustable aperture 4. Alternatively, the outer sheet of the adjustable aperture may be the housing itself. This allows the humidity and temperature within the mask housing 2 to be selectively controlled.

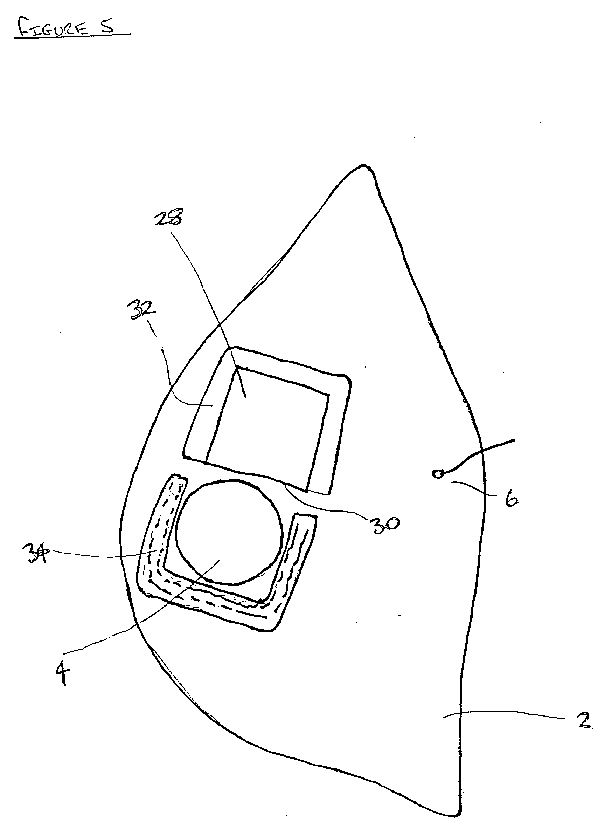

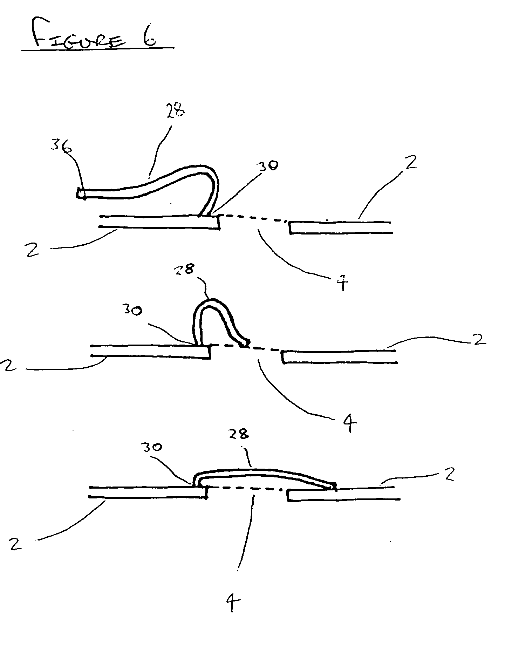

Figure 5 is a schematic side view of the second exemplary embodiment of the mask arrangement, showing a further embodiment of the adjustable aperture. A simple flexible flap 28 is shown, at one edge hingedly attached 30 to the housing 2. An adhesive material, such as Velcro (RTM) 32 is adhered to three edges of the flap 28, and corresponding material 34 is adhered to the corresponding area located surrounding three sides of the aperture 4 on the housing 2. In use, as is visible in the side view of the Figure 5 as shown in Figure 6, the flap can be adjusted to control the humidity within the housing 2 by folding the flap 28 back to the desired degree. If the flap 28 is to be fully open, an adhesive means can be provided on the outer surface 36 of the flap, such that it stays in position in use. Accordingly, the flap can be fully open or fully closed, and is adjustable to any degree in between these parameters. In order that the mask is safe, the mask material or at least part of it should be of a suitably porous material or contains small perforations such that it is impossible to be smothered by the mask. The pores or perforations in the mask material are sufficient for safety purposes whilst not affecting the functioning of the mask.

The fastening mechanism may be any type as indicated above, but in the simplest embodiment comprises one or more adjustable elastic straps which wrap around the user's head. The fastening mechanisms are sufficiently elastic or of a sufficiently low breaking strain that they cannot cause accidental choking to a user if caught on something, especially when a user is asleep.

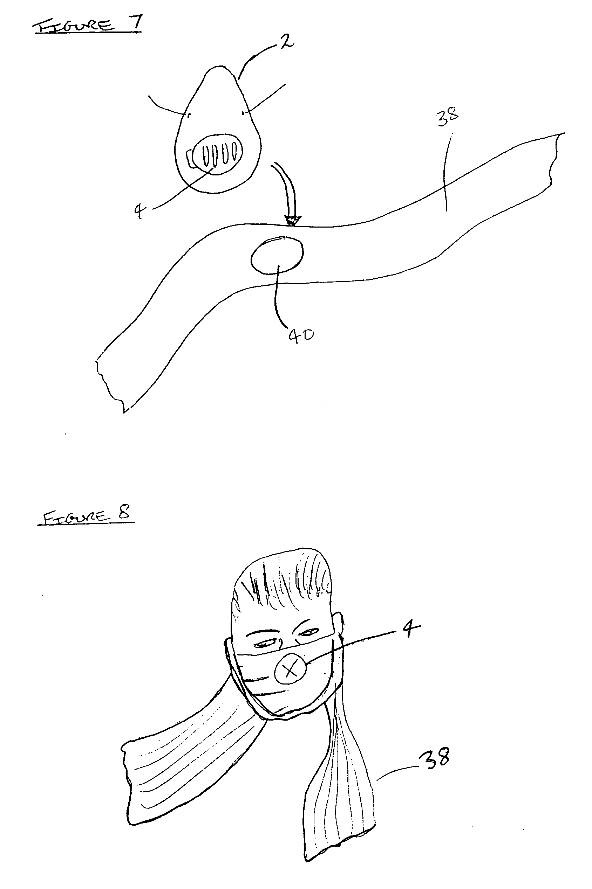

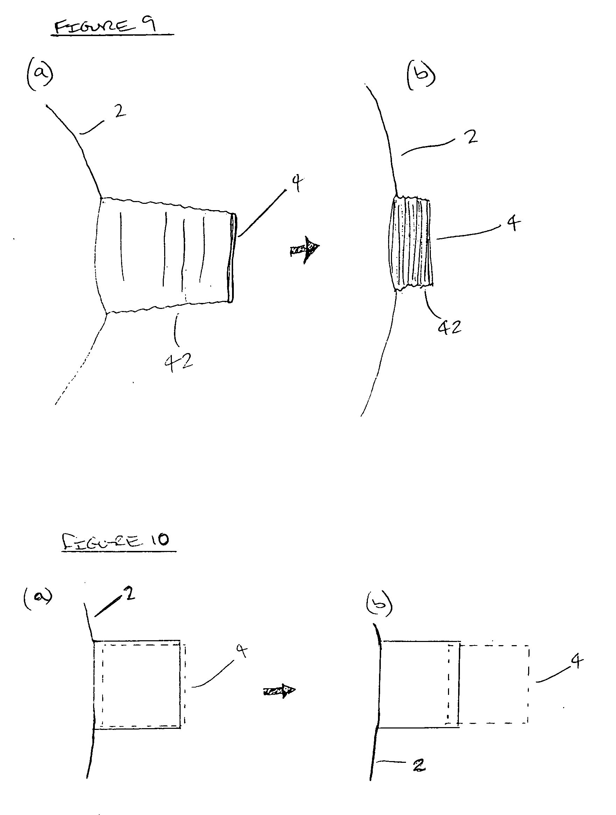

Referring to Figure 7 and 8, the mask arrangement may be utilised integrally with or in combination with standard items of clothing such as scarves 38 or balaclavas. In a simplest embodiment, an aperture 40 is provided in a scarf 38 which enables a mask to protrude therethrough. This may be particularly beneficial for fashionable wearing of a mask.

In a further embodiment as indicated in Figure 8, the mask is joined integrally with a scarf 38. In this case, the fastening means as indicated in previous embodiments is replaced by the scarf itself. The mask may be attached to the inner face of the scarf by any known means such as sewing or adhesives. The rigidity of the housing is maintained to ensure continued function of the mask.

In a further embodiment of the present invention, the size of the cavity within the mask is adjustable. In a first embodiment as indicated in Figure 9, a bellows 42 type structure protrudes from the housing 2. The adjustable aperture is mounted in the bellows 42. Utilising this arrangement means a user can adjust the size of the cavity inside the housing 2 by pushing or pulling. Figure 9a indicates the bellow 42 in the extended position and 9b in the reduced cavity volume position.

Referring to Figure 10, in an alternative embodiment to that shown in Figure 9, the aperture 4 may be mounted on a telescopic section, indicated as in the reduced cavity section (Figure 10a) and the extended cavity (Figure 10b), which increases the cavity volume.

A distinct advantage of the present invention is that there is no requirement for the air to be filtered before being breathed in by the user, therefore the user can breathe without restriction. A further advantage of the present invention is that the mask allows almost normal speech due to the apertures enabling sound to pass without significant impedance. This advantage, combined with there being no need for the mask to be attached to any external apparatus or connections, makes almost normal ambulatory and conversational activity possible.

In any of the described embodiments, the adjustable aperture may be shaped and configured to receive an approved device to administer inhalable agents such as oxygen or nebulised medication. Alternatively, a second aperture may be specifically provided for this purpose.

Embodiments of a face mask according to the present invention have been described by way of example only and it will be appreciated by a person skilled in the art that variations and modifications can be made in respect of the described embodiments without departing from the scope of the invention as defined in the appended claims.

## Claims

1. A face mask comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, said face mask including means to adjust the size of said aperture to control the air flow into and out of said housing, such that, in use, the humidity and/or temperature of the air within said housing may be controlled.

2. A mask according to claim 1 wherein said housing is arranged and configured to fit over a user's nose and mouth.

3. A mask according to claims 1 or 2 wherein the means to adjust the size of said aperture comprises overlapping slats, and/or comprises at least one flexible flap.

4. A mask according to claim 1 wherein the means to hold said housing adjacent a user's face comprises at least one resiliently elastic strap.

5. A mask according to any of claims 1-2 or 4 wherein said housing is constructed of a flexible material.

6. A mask according to any of claims 1-2 or 4 wherein said housing is constructed of a substantially rigid material.

7. A mask according to any preceding claim further comprising means to adjust the volume of air within said housing.

8. A mask according to Claim 7, wherein said volume adjusting means comprising a bellows structure, and/or a telescopic arrangement.

9. A mask according to any preceding claim, further comprising a second aperture in said housing, arranged and configured to receive means to provide a gas into said cavity.

10. Headwear comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, said face mask including means to adjust the size of said aperture to control the air flow into and out of said housing, such that, in use, the humidity and/or temperature of the air within said housing may be controlled.

11. A face mask, when in use for prevention of one or more symptoms of nose and/or mouth breathing, the face mask comprising a housing being arranged and configured to fit over at least a user's mouth, said housing defining, in use, a hollow space between at least a user's mouth and the inner surface of the housing, further comprising means for removably holding said housing adjacent to at least a user's mouth, said housing further containing an aperture therein, such that, in use, the humidity and/or temperature of the air within said housing may be increased relative to the surrounding atmosphere.

12. A face mask according to claim 11 wherein said face mask includes means to adjust the size of the aperture to control the air flow into and out of said housing such that, in use, the humidity and/or temperature of the air within the housing may be controlled.

13. A method of preventing one or more symptoms of nose and/or mouth breathing, the method comprising the steps of providing a face mask comprising a housing being arranged and configured to fit over at least a user's mouth, said housing defining, in use, a hollow space between at least a user's mouth and the inner surface of the housing, further comprising means for removably holding said housing adjacent at least a user's mouth, said housing further containing an aperture therein, such that, in use, the humidity and/or temperature of the air within said housing may be increased relative to the surrounding atmosphere.

14. A method according to claim 13, wherein said face mask includes means to adjust the size of said aperture to control the air flow into and out of said housing, such that, in use, the humidity and/or temperature of the air within said housing may be controlled.
